# EUROPEAN PATENT APPLICATION

(11) **EP 4 383 266 A1**
(43) Date of publication of application: **12.06.2024**
(21) Application number: 22212003.2
(22) Date of filing: 07.12.2022
(51) Int. Cl.: G16H 10/60

(54) **METHODS AND SYSTEMS FOR TRANSMITTING MEDICAL INFORMATION ACCORDING TO PRIORITIZATION CRITERIA**

(71) Applicant: Koninklijke Philips N.V., 5656 AG Eindhoven (NL)
(72) Inventor: STUT, Wilhelmus Johannes Joseph, Eindhoven (NL); HUIJGEN, Henk, Eindhoven (NL); BRANDSMA, Ewout, 5656AG Eindhoven (NL)
(74) Representative: Philips Intellectual Property & Standards

(57) **Abstract**

The present disclosure relates to remote physiological monitoring. More specifically, provided herein are methods and systems for prioritizing transmission of physiological data collected from a patient where the sensor and/or system comprising the sensor does not have consistent wireless connectivity. In particular, a mobile physiological data telemetry system for prioritizing transmission of physiological monitoring data is provided that comprises: a physiological sensor; a communication interface, a set of prioritization rules for the patient; and a processor configured to identify one or more portions of the physiological data and prioritize the portions according to the prioritization rules for the patient.

## Description

### Field of the Disclosure

The present disclosure relates generally to methods and systems for remotely monitoring a subject, and more specifically to mobile physiological data telemetry systems and methods of prioritizing transmission of physiological data.

### Background

The availability of different wireless communication networks has enabled the development of remote physiological monitoring. The field of remote physiological monitoring relates to the remote sensing of a person's vital signs. Remote physiological monitoring also has applications in sports programs and physical therapy programs, and in other settings, such as monitoring individuals in hazardous environments. However, gaps in network coverage connecting a remote physiological monitoring device to a backend receiver have led to significant challenges in reporting accurate vital sign measurements in a timely and clinically-efficient manner.

### Summary of the Disclosure

According to an embodiment of the present disclosure, a method for prioritizing transmission of physiological monitoring data is provided. The method comprises: providing a mobile physiological data telemetry system, comprising a physiological sensor configured to receive physiological data from a patient, and further comprising a communication interface configured to connect to a communications network, wherein the mobile physiological data telemetry system comprises a set of prioritization rules for the patient; receiving, from the physiological sensor, physiological data from the patient; determining, by the mobile physiological data telemetry system, that a first portion of the received physiological data is identified for transmission via the communication interface; determining, by the mobile physiological data telemetry system, that a second portion of the received physiological data is identified for transmission via the communication interface; prioritizing, when the mobile physiological data telemetry system is not connected to the communications network, and using the set of prioritization rules, transmission of the first portion before the second portion of the received physiological data, or vice versa; and transmitting, when the mobile physiological data telemetry system is connected to the communications network, the first portion and the second portion of the received physiological data in the prioritized order.

In an aspect, the mobile physiological data telemetry system is a wireless physiological sensor device attached to the patient.

In an aspect, the mobile physiological data system comprises a smartphone, the smartphone comprising the wireless communication interface.

In an aspect, determining that the first portion of the received physiological data and the second portion of the received physiological data are identified for transmission is based on a parameter of the received physiological data.

In an aspect, prioritization using the set of prioritization rules is based on a parameter of the received physiological data.

In an aspect, the method further comprises the step of receiving, by the mobile physiological data telemetry system, the set of prioritization rules for the patient.

In an aspect, the set of prioritization rules are specific to a particular patient.

In an aspect, the method further includes determining, using the set of prioritization rules, that a third portion of the received physiological data is identified for transmission via the communication interface; wherein the step of prioritizing comprises prioritizing transmission of the first portion, the second portion, and the third portion in a predetermined order of priority; and wherein the step of transmitting comprises transmitting the first portion, the second portion, and the third portion in the predetermined order of priority.

According to another embodiment of the present disclosure, a mobile physiological data telemetry system for prioritizing transmission of physiological monitoring data is provided. The mobile physiological data telemetry system comprises: a physiological sensor configured to receive physiological data from a patient; a communication interface configured to connect to a communications network; a set of prioritization rules for the patient; a processor configured to: (i) receive, from the physiological sensor, physiological data from the patient; (ii) determine, by the mobile physiological data telemetry system, that a first portion of the received physiological data is identified for transmission via the communication interface; (iii) determine, by the mobile physiological data telemetry system, that a second portion of the received physiological data is identified for transmission via the communication interface; (iv) prioritize, when the mobile physiological data telemetry system is not connected to the communications network, and using the set of prioritization rules, transmission of the first portion before the second portion of the received physiological data, or vice versa; and (v) transmit, via the communication interface when the mobile physiological data telemetry system is connected to the communications network, the first portion and the second portion of the received physiological data in the prioritized order.

In an aspect, the mobile physiological data telemetry system comprises a wireless physiological sensor device attached to the patient.

In an aspect, the mobile physiological data telemetry system comprises a smartphone, the smartphone comprising the wireless communication interface.

In an aspect, determining that the first portion of the received physiological data and the second portion of the received physiological data are identified for transmission is based on a parameter of the received physiological data.

In an aspect, prioritization using the set of prioritization rules is based on a parameter of the received physiological data.

In an aspect, the set of prioritization rules are specific to a particular patient.

In an aspect, the processor is further configured to determine, using the set of prioritization rules, that a third portion of the received physiological data is identified for transmission via the communication interface; wherein prioritizing comprises prioritizing, based on the set of prioritization rules, transmission of the first portion, the second portion, and the third portion in a predetermined order of priority; and wherein transmitting comprises transmitting the first portion, the second portion, and the third portion in the predetermined order of priority.

These and other aspects of the various embodiments will be apparent from and elucidated with reference to the embodiments described hereinafter.

### Brief Description of the Drawings

In the drawings, like reference characters generally refer to the same parts throughout the different views. Also, the drawings are not necessarily to scale, emphasis instead generally being placed upon illustrating the principles of the various embodiments.
FIG. 1 is a flowchart of a method for prioritizing transmission of physiological monitoring data illustrated according to aspects of the present disclosure.
FIG. 2 is a block diagram of a mobile physiological data telemetry system illustrated according to aspects of the present disclosure.
FIG. 3A is a schematic block diagram illustrating a controller used in a mobile physiological data telemetry system according to aspects of the present disclosure.
FIG. 3B is a block diagram illustrating a portion of the controller of FIG. 3A.
FIG. 4 is an illustration showing the chronological collection of physiological data according to aspects of the present disclosure.
FIG. 5 is a diagram illustrating the operational use of a mobile physiological data telemetry system according to aspects of the present disclosure.

### Detailed Description of Embodiments

The present disclosure is directed to mobile physiological data monitoring systems, which is a field of remote physiological monitoring that involves gathering physiological data in ambulatory settings. More specifically, the systems of the present disclosure provide a direct-to-internet device that includes electronics for wirelessly communicating physiological data to a backend server, and which is configured to prioritize and/or selectively transmit physiological data based on customizable prioritization criteria.

In accordance with an embodiment, the present disclosure is directed to mobile cardiac outpatient telemetry (MCOT) systems, which is a field of remote physiological monitoring that involves gathering electrocardiogram (ECG) data in ambulatory settings. More specifically, the MCOT systems of the present disclosure provide a direct-to-internet device that includes electronics for wirelessly communicating ECG data to a backend server, and which is configured to prioritize and/or selectively transmit ECG data based on customizable prioritization criteria. In addition to or alternative to ECG data, the mobile physiological data monitoring systems may obtain other types of physiological data using one or more physiological sensors in the mobile system, including but not limited to PPG data (including many different aspects of PPG data), respiratory rate, temperature, apnea, movement, and many other types of physiological data.

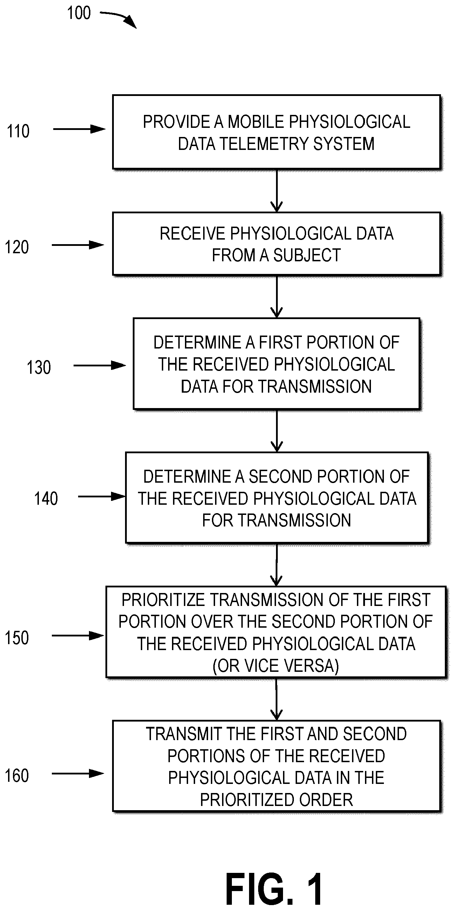

Turning to FIG. 1, a method 100 for prioritizing transmission of physiological monitoring data is provided according to aspects of the present disclosure. In embodiments, the method 100 includes: in a step 110, providing a mobile physiological data telemetry system; in a step 120, receiving from a physiological sensor of the mobile physiological data telemetry system, physiological data from a subject; in a step 130, determining that a first portion of the received physiological data is identified for transmission via the communication interface; in a step 140, determining that a second portion of the received physiological data is identified for transmission via the communication interface; in a step 150, prioritizing intended transmission of the first portion before the second portion of the received physiological data, or vice versa, when the mobile physiological data telemetry system is not connected to a communications network based on the prioritization rules; and, in a step 160, transmitting the first portion and the second portion of the received physiological data in the prioritized order when the mobile physiological data telemetry system is reconnected to the communications network.

In accordance with an embodiment, FIG. 1 is a method 100 for prioritizing transmission of ECG monitoring data is provided according to aspects of the present disclosure. In embodiments, the method 100 includes: in a step 110, providing a mobile cardiac telemetry system; in a step 120, receiving from an electrocardiogram (ECG) sensor of the mobile cardiac telemetry system, ECG data from a subject; in a step 130, determining that a first portion of the received ECG data is identified for transmission via the communication interface; in a step 140, determining that a second portion of the received ECG data is identified for transmission via the communication interface; in a step 150, prioritizing intended transmission of the first portion before the second portion of the received ECG data, or vice versa, when the mobile cardiac telemetry system is not connected to a communications network based on the prioritization rules; and, in a step 160, transmitting the first portion and the second portion of the received ECG data in the prioritized order when the mobile cardiac telemetry system is reconnected to the communications network.

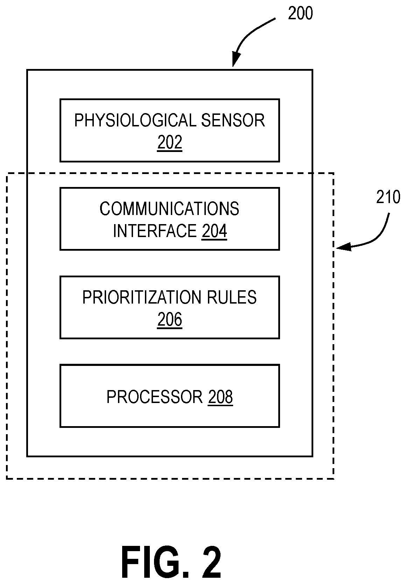

More specifically, in the step 110, the method 100 includes providing a mobile cardiac telemetry system that comprises: an ECG sensor configured to receive ECG data from a subject; a communication interface configured to connect the mobile cardiac telemetry system to a communications network; and a set of prioritization rules for the subject. For example, with reference to FIG. 2, an exemplary mobile physiological data telemetry system 200 is illustrated according to aspects of the present disclosure. In this embodiment, the physiological data telemetry system is a mobile cardiac telemetry system, although as described or otherwise envisioned herein, the mobile physiological data telemetry system may be one of many other types of physiological data systems. As shown, the mobile cardiac telemetry system 200 comprises an ECG sensor 202, a communications interface 204, a set of prioritization rules 206, and one or more processors 208. In embodiments, the ECG sensor 202 is configured to receive ECG data from a subject, the communications interface 204 is configured to connect the system 200 to a communications network, and the one or more processors 208 are configured to perform one or more steps of the methods described herein.

In embodiments, the system 200 is a wireless ECG sensor device which is attached to the body of the subject and is entirely mobile with the subject. In some embodiments, the system 200 comprises a mobile electronic device having a communications interface 204, including but not limited to, a laptop, a personal digital assistant (PDA), mobile phone, a smartphone, a wearable computing and communicating device, and/or the like.

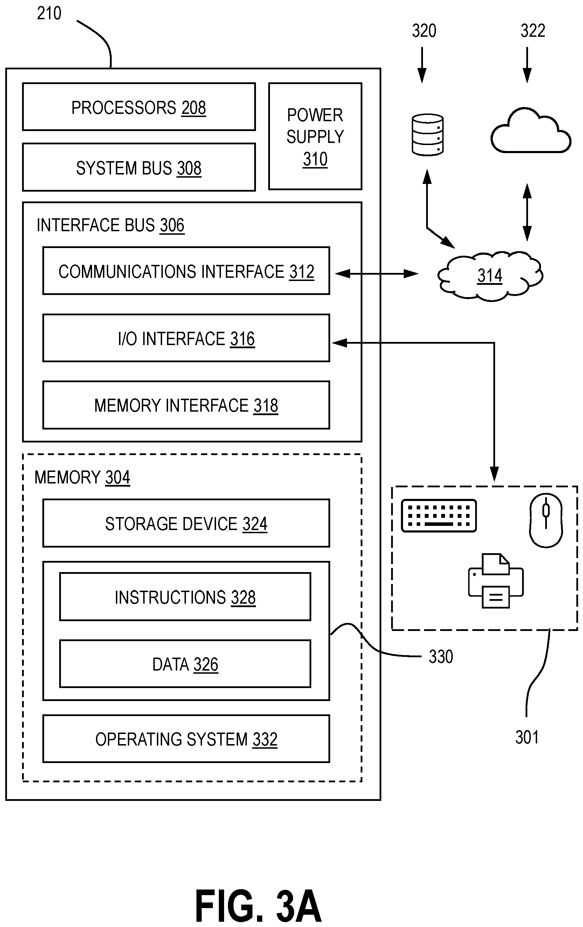

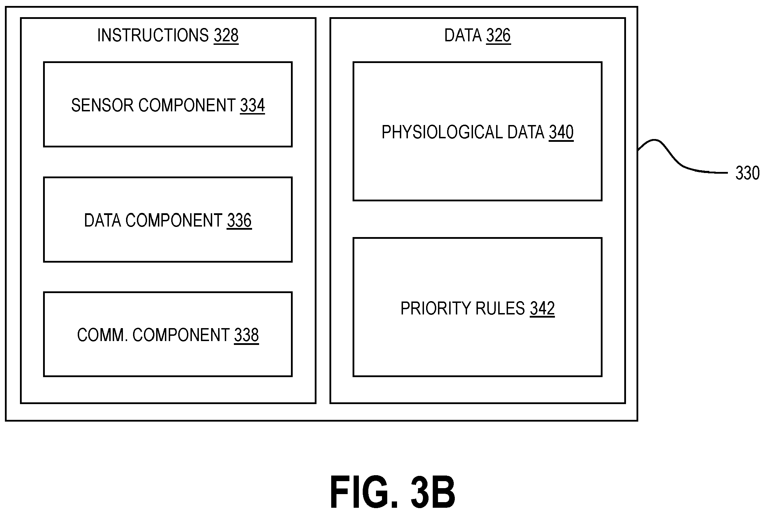

In particular embodiments, the system 200 may include a controller 210 comprising one or more of the communications interface 204, the prioritization rules 206, and/or the one or more processors 208. With reference to FIGS. 3A and 3B, a block diagram of a controller 210 that may form a part of the system 200 is illustrated. The controller 210 may be operatively connected to one or more other components of the system 200, including but not limited to the ECG sensor 202 and/or the mobile electronic device (not shown). The controller 210 may be configured to operate the ECG sensor 202, the communications interface 204, and/or implement the prioritization rules 206 as described herein.

In the example of FIG. 3A, the controller 210 can include the one or more processors 208, machine-readable memory 304, and an interface bus 306, all of which may be interconnected and/or communicate through a system bus 308 containing conductive circuit pathways through which instructions (e.g., machine-readable signals) may travel to effectuate communication, tasks, storage, and the like. The controller 210 may be connected to a power source 310, which can include an internal power supply and/or an external power supply.

The one or more processors 208 may include a high-speed data processor adequate to execute the program components described herein and/or various specialized processing units as may be known in the art. In some examples, the one or more processors 208 may be a single processor, multiple processors, or multiple processor cores on a single die.

In some examples, the interface bus 306 may include a communications interface 304 configured to connect the system 200 to a communications network 314, an input/output ("I/O") interface 316 configured to connect and communicate with one or more peripheral devices 301, and/or a memory interface 318 configured to accept, communication, and/or connect to a number of machine-readable memory devices (e.g., memory 304).

The I/O interface 316 may operatively connect the controller 210 and/or the system 200 with one or more peripheral devices 301. In some examples, the peripheral devices 301 may include, but are not limited to, display monitors; joysticks; keyboards; microphones; computer mouse (mice); touch screens (e.g., capacitive, resistive, etc.); trackballs; trackpads; styluses; audio devices; cameras; printers; video devices; and/or the like.

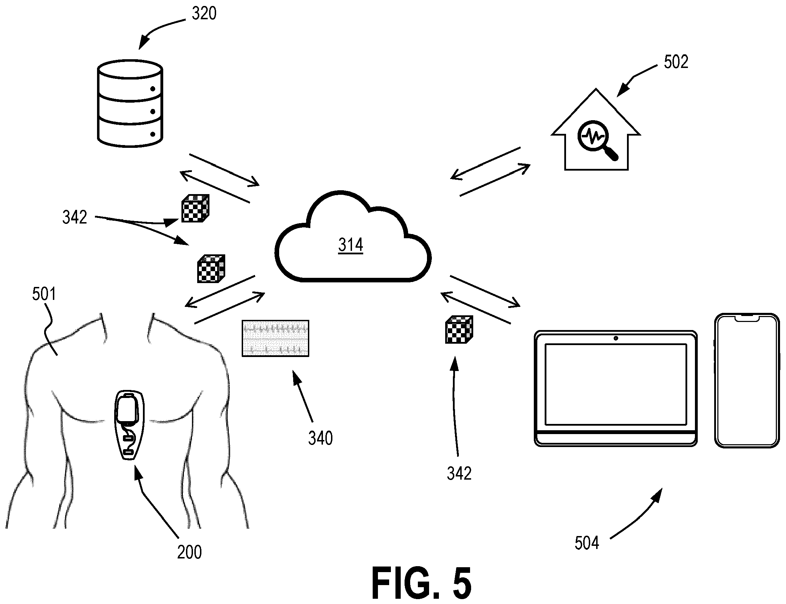

The communications interface 312 may operatively connect the controller 210 and/or system 200 to a communications network 314, which can include a direct interconnection, the Internet, a local area network ("LAN"), a metropolitan area network ("MAN"), a wide area network ("WAN"), a wired or Ethernet connection, a wireless connection, and similar types of communications networks, including combinations thereof. In further embodiments, the communications network 314 can include other communication means, such as Bluetooth, cellular data networks (e.g., 3G, 4G, 5G, LTE, etc.), or the like. In some examples, controller 210 and/or system 200 may communicate with one or more remote / cloud-based servers 320, cloud-based services 322, and/or remote devices (e.g., device 504 shown in FIG. 5) via the communications network 314 and the communications interface 204.

The memory 304 can be variously embodied in one or more forms of machine-accessible and machine-readable memory. In some examples, the memory 304 includes a storage device 324 comprises one or more types of memory. For example, the storage device 324 can include, but is not limited to, a non-transitory storage medium.

Generally, the memory 304 is configured to store data / information 326 and instructions 328 that, when executed by the one or more processors 208, causes the controller 210 and/or system 200 to perform one or more tasks.

In particular examples, the memory 304 includes a mobile cardiac telemetry package 330 that comprises a collection of program components, database components, and/or data. Depending on the particular implementation, the mobile cardiac telemetry package 330 may include software components, hardware components, and/or some combination of both hardware and software components.

The mobile cardiac telemetry package 330 may include, but is not limited to, instructions 328 having one or more software packages. These software packages may be incorporated into, loaded from, loaded onto, or otherwise operatively available to and from the controller 210 and/or system 200. Put another way, the mobile cardiac telemetry package 330 and/or one or more software packages may be incorporated into, loaded from, loaded onto, or otherwise operatively available to a mobile cardiac telemetry system 200.

In some examples, the mobile cardiac telemetry package 330 and/or one or more individual software packages may be stored in a local storage device 324. In other examples, the mobile cardiac telemetry package 330 and/or one or more individual software packages may be loaded onto and/or updated from a remote server 320 via the communications network 314.

In particular embodiments, the mobile cardiac telemetry package 330 can include, but is not limited to, instructions 328 having a sensor component 334, a data component 336, and/or a communications component 338. These components may be incorporated into, loaded from, loaded onto, or otherwise operatively available to and from the system 200.

The sensor component 334 can be a stored program component that is executed by at least one processor, such as the one or more processors 208 of the system 200. In particular, the sensor component 334 can be configured to receive or otherwise obtain ECG sensor data 340, as described herein. In embodiments, the sensor component 334 can be configured to store one or more portions of the received ECG sensor data 340 in a memory 304. For example, one or more portions of the received ECG sensor 340 data may be stored in memory 304 while the system 200 is unable to connect to a communications network 314, if the system 200 is running low on power, and/or when it is otherwise undesirable to immediately transmit the ECG sensor data 340.

The data component 336 can be a stored program component that is executed by at least one processor, such as the one or more processors 208 of the system 200. In particular, the data component 336 can be configured to identify one or more subsets of the ECG sensor data 340, as described herein. For example, the data component 336 can be configured to determine, using any predetermined programming or rules, that at least a first portion of the received ECG data 340 is identified for transmission via the communications interface 204. In embodiments, the data component 336 can be configured to determine, using the predetermined programming or rules, that at least a second portion of the received ECG data 340 is identified for transmission via the communications interface 204.

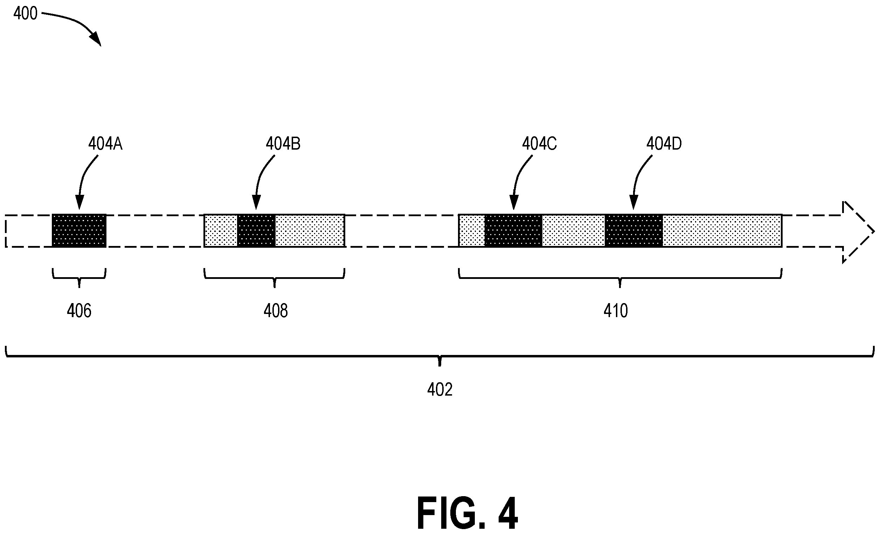

For example, with reference to FIG. 4, a chronological representation 400 of ECG sensor data 340 collection is illustrated over a period of time 402 according to aspects of the present disclosure. The system 200 may collected one or more subsets 406, 408, 410 of ECG data 340 as described herein. As shown, the system 200 may only collect ECG data 340 at certain times or upon detecting certain conditions. From each of the subsets 406, 408, 410 of ECG data 340, the system 200 may determine that one or more portions 404A, 404B, 404C, 404D of the ECG data 340 should be transmitted for analysis and/or reporting purposes. As shown, each of the one or more portions 404A, 404B, 404C, 404D of the ECG data 340 may correspond to a period of time in which ECG data 340 was being collected (e.g., subsets 406, 408, 410). In particular, it should be appreciated that the one or more portions 404A, 404B, 404C, 404D of the ECG data 340 may cover a period of time that is less than the entire instance of ECG data 340 collection.

According to an embodiment, the system 200 may be preprogrammed with rules, triggers, or thresholds, or other indicators that allow the system to determine when ECG sensor data should be transmitted or otherwise shared or communicated for analysis and/or reporting purposes. The rules, triggers, thresholds, or other indicators may be preprogrammed into the system as a default, may be provided to the system by a clinician or other individual, and/or may be designed for a particular person and/or setting. Many other variables are possible. According to an embodiment, in addition to prioritization rules, the system 200 may be programmed with notification criteria or rules that enable the system to determine what ECG sensor data should be - and/or should not be - transmitted or otherwise shared or communicated for analysis and/or reporting purposes. These notification criteria or rules can be preprogrammed into the system as a default, may be provided to the system by a clinician or other individual, and/or may be designed for a particular person and/or setting.

In particular embodiments, the system 200 (e.g., the data component 336) may determine a priority list for transmitting the one or more portions 404A, 404B, 404C, 404D of the ECG data 340. For example, instead of transmitting the one or more portions 404A, 404B, 404C, 404D of the ECG data 340 in chronological order (from earliest to latest or from latest to earliest), it may be determined, based on a set of prioritization rules 342, to prioritize transmission of the one or more portions 404A, 404B, 404C, 404D of the ECG data 340 is non-chronological order. These prioritization rules or criteria 342 may be preprogrammed into the system as a default, may be provided to the system by a clinician or other individual, and/or may be designed for a particular person and/or setting. According to one embodiment, the prioritization rules 342 may be the same as or based on notification criteria or rules, although many other criteria may be utilized.

In embodiments, the priority of the one or more subsets of ECG sensor data 340 may be based upon when the system 200 is or is not connected to the communications network 314, has insufficient power, or when it is otherwise undesirable to perform a data transmission operation. For example, when the battery level has fallen below a predefined battery level threshold, the prioritization rules may indicate that the system should prioritize and/or only transmit the one or more subsets of ECG sensor data 340 with a high notification and/or prioritization score (such as above a predefined notification and/or prioritization threshold). At a later time, when the battery has been fully or partly recharged, the remaining subsets of ECG sensor data 340 with a low notification and/or prioritization score (such as below the predefined notification and/or prioritization threshold) can be transmitted.

The communications component 338 can be a stored program component that is executed by at least one processor, such as the one or more processors 208 of the system 200. In particular, the communications component 338 can be configured to transmit one or more subsets of ECG sensor data 340 based on the prioritization rules 342 via the communications interface 204/304 and/or the communications network 314.

With further reference to FIG. 5, the one or more subsets of ECG sensor data 340 received from a subject or patient 501 may be transmitted to, for example, a remote server 320, a clinical service center 502, and/or a remote device 504 over a communications network 314. In embodiments, the one or more subsets of ECG sensor data 340 received at the remote server 320 may be stored in a memory of the remote server 320. In further embodiments, one or more subsets of transmitted ECG sensor data 340 may be received at the clinical service center 502 and analyzed by software and/or ECG technicians at the clinical service center 502. In still further embodiments, one or more subsets of transmitted ECG sensor data 340 may be accessed and/or reviewed by a healthcare specialist (e.g., a physician, clinician, nurse, etc.) via the remote device 504.

In embodiments, the communications component 338 can also be configured to receive the prioritization rules 342 via the communications interface 204 and the communications network 314. For example, a healthcare specialist (e.g., a physician, clinician, nurse, etc.) may provide prioritization criteria 342 via a remote device 504, for example, which may then be stored at a remote server, such as remote server 320. The prioritization criteria may be stored at the remote server, from which these can be downloaded to, or uploaded by the device.

In embodiments, the prioritization criteria 342 provided by the healthcare specialist may be determined based on a general profile for the patient wearing the ECG sensor device 200. For example, the general profile can include details such as the patient's medical history, diagnoses, treatment history, and/or the like. In particular embodiments, the prioritization criteria 342 are specific to the patient.

According to the present disclosure, the prioritization rules 342 can include a number of different criteria. For example, in some embodiments, the prioritization criteria 342 can include parameters such as heart rate. In particular embodiments, the prioritization criteria 342 can include detecting conditions that last a predetermined period of time. For example, in some embodiments, the prioritization criteria 342 can include determining whether the patient's heart rate was above a predetermined threshold for a predetermined period of time. Based on the detection of the condition, the prioritization criteria 342 may determine a priority for the detected event, which then determines the order in which the ECG data 340 is transmitted.

The controller 210 may also include an operating system component 332, which may be stored in the memory 304. The operating system component 332 may be an executable program facilitating the operation of the controller 210. Typically, the operating system component 332 can facilitate access of the I/O interface, communications interface, and memory interface, and can communicate with other components of the system 200.

Returning to FIG. 1, the method 100 can include, in the step 120, receiving ECG data 340 from a patient or subject from the ECG sensor 202. As discussed with respect to FIG. 4, ECG data 340 may be received from the patient or subject via the ECG sensor 202 during particular periods of time. In other embodiments, ECG sensor data 340 may be collected continuously.

In the step 130, the method 100 includes determining that a first portion of the received ECG data 340 is identified for transmission via the communications interface 204. In embodiments, the one or more processors 208 of the system 200 may be configured to determine that a first portion of the received ECG data 340 is identified for transmission. As described herein, the first portion of the received ECG data 340 can be, for example, at least one of portions 404A, 404B, 404C, 404D of the ECG data 340.

In the step 140, the method 100 includes determining that a second portion of the received ECG data 340 is identified for transmission via the communications interface 204. In embodiments, the one or more processors 208 of the system 200 may be configured to determine that a second portion of the received ECG data 340 is identified for transmission. As described herein, the second portion of the received ECG data 340 can be, for example, at least one of portions 404A, 404B, 404C, 404D of the ECG data 340, but is different from the first portion of the received ECG data 340.

Although steps 130 and 140 describe determining that a first portion and a second portion of the ECG data 340 is identified for transmission via the communications interface 204, any number of portions may be ECG data 340 is identified for transmission via the communications interface. For example, the method may include additional steps 140 in which a third portion of the received ECG data is identified for transmission via the communication interface, a fourth portion of the received ECG data is identified for transmission via the communication interface, and so on.

In the step 150, the method 100 includes prioritizing transmission of at least one of the first and second portions of the received ECG data 340. As described herein, the prioritization of the ECG data 340 can include determining an order in which each portion 404A, 404B, 404C, 404D of the received ECG data 340 should be transmitted. In embodiments, the one or more processors 208 of the system 200 may be configured to prioritize transmission of at least one of the first and second portions of the received ECG data 340. In particular embodiments, the one or more portions 404A, 404B, 404C, 404D of the received ECG data 340 may be prioritized when the system 200 is unable to connect to a communications network 314 or is otherwise unable to transmit ECG data 340. For example, in some embodiments, the system 200 may have a low-power operating mode in which power must be conserved, leading to an inability to transmit large amounts of ECG data 340.

In some embodiments, the one or more processors 208 of the system 200 may be configured to prioritize transmission of any number of portions of the received ECG data, including but not limited to the first portion and second portion as well as a third portion, a fourth portion, and so on.

In the step 160, the method 100 includes transmitting one or more portions 404A, 404B, 404C, 404D of the received ECG data 340 in the prioritized order. In embodiments, the one or more processors 208 of the system 200 may be configured to transmit the one or more portions 404A, 404B, 404C, 404D of the received ECG data 340 in the prioritized order, when the system is reconnected to the communications network. As described herein, one or more portions 404A, 404B, 404C, 404D of the received ECG data 340 may be sent to a clinical service center 402 where an ECG technician may review and/or analyze the one or more portions 404A, 404B, 404C, 404D of the received ECG data 340 for certain parameters or conditions. In embodiments, once one or more portions 404A, 404B, 404C, 404D of the received ECG data 340 are verified, the healthcare specialist may be notified in accordance with patient-specific notification criteria.

In some embodiments, each cardiac event represented in one or more portions 404A, 404B, 404C, 404D of the received ECG data 340 may be added to a report. That is, in some embodiments, the method 100 can include generating a report based on the one or more portions 404A, 404B, 404C, 404D of the received ECG data 340 that identifies one or more cardiac events for the patient. In further embodiments, the method 100 can include receiving, at the system 200, a set of prioritization rules 342 for the patient 501. In some embodiments, the healthcare specialist may establish the set of prioritization rules 342 when prescribing use of the monitoring system 200, and the system 200 may receive the set of prioritization rules 342 prior to obtaining ECG data 340 from the patient 501 via the ECG sensor 202.

According to an embodiment, after sending an ECG portion with the highest priority, the system 200 can communicate a message with a subset of the information about the one or more additional ECG portions that have been prioritized for transmission but have not yet been transmitted. The subset of information could include one or more characteristics about the ECG portion(s) (e.g., a timestamp, and/or clinical parameters such as the arrhythmia type (atrial fibrillation or atrial flutter)). This information may be useful for the ECG technician when processing the initial transmitted ECG portion with the highest priority. Accordingly, the prioritization rules could include thresholds, programming, or other information directing the system 200 which subset of information to send about a prioritized ECG portion, which prioritized ECG portion a subset of information should be sent about, and other details of transmission.

According to an embodiment, at step 160 of the method, after the system determines that it is reconnected to the communications network and before transmitting one or more portions 404A, 404B, 404C, 404D of the received ECG data 340 in the prioritized order, the system can contact the cloud or otherwise receive additional recent data on the patient. In particular, when the prioritization criteria refer to data that is not collected by the system (e.g. symptom information such as "the patient felt dizzy"), these data can be sent to the system. This additional data may change the order in which the ECG strips are sent, by modifying the prioritization rules and thus changing the order of the one or more portions 404A, 404B, 404C, 404D. Thus, the system can receive this additional data, which could also be a modification of the prioritization rules by a clinician, and thus return to step 150 to re-prioritize the transmission of the ECG data portions, and at step 160 can transmit those re-prioritized ECG data portions.

Notably, according to another embodiment, the methods and systems described or otherwise envisioned herein can be utilized for prioritization and communication of other types of data, including but not limited many different types of physiological data. This can include patient sensor data such as PPG data and many other types of data. Thus, in accordance with an embodiment, a remote, mobile, or otherwise transmitting system could comprise: a physiological data sensor configured to receive physiological data from a subject; a communication interface configured to connect the physiological data system to a communications network; and a set of prioritization rules for the system and/or the subject. For example, in reference to an embodiment of FIG. 2, a physiological data system 200 could comprise a physiological sensor 202, a communications interface 204, a set of prioritization rules 206, and one or more processors 208. In embodiments, the physiological sensor 202 is configured to receive physiological data from a subject, the communications interface 204 is configured to connect the system 200 to a communications network, and the one or more processors 208 are configured to perform one or more steps of the methods described herein. Referring to an embodiment of FIG. 1, a method 100 for prioritizing transmission of physiological data comprises: in a step 110, providing a physiological data system; in a step 120, receiving from a physiological sensor of the system, physiological data from a subject; in a step 130, determining that a first portion of the received physiological data is identified for transmission via the communication interface; in a step 140, determining that a second portion of the received physiological data is identified for transmission via the communication interface; in a step 150, prioritizing intended transmission of the first portion before the second portion of the received physiological data, or vice versa, when the physiological data system is not connected to a communications network based on the prioritization rules; and, in a step 160, transmitting the first portion and the second portion of the received physiological data in the prioritized order when the physiological data system is reconnected to the communications network.

In accordance with such an embodiment, a remote, mobile, or otherwise transmitting PPG system could comprise: a PPG sensor configured to receive PPG data from a subject (such as heart rate, oxygen saturation, respiration rate, blood pressure, etc.); a communication interface configured to connect the PPG system to a communications network; and a set of prioritization rules for the subject. For example, in reference to an embodiment of FIG. 2, a PPG system 200 could comprise a PPG sensor 202, a communications interface 204, a set of prioritization rules 206, and one or more processors 208. In embodiments, the PPG sensor 202 is configured to receive PPG data from a subject, the communications interface 204 is configured to connect the system 200 to a communications network, and the one or more processors 208 are configured to perform one or more steps of the methods described herein. Referring to an embodiment of FIG. 1, a method 100 for prioritizing transmission of PPG data comprises: in a step 110, providing a PPG system; in a step 120, receiving from a PPG sensor of the PPG system, PPG data from a subject; in a step 130, determining that a first portion of the received PPG data is identified for transmission via the communication interface; in a step 140, determining that a second portion of the received PPG data is identified for transmission via the communication interface; in a step 150, prioritizing intended transmission of the first portion before the second portion of the received PPG data, or vice versa, when the PPG system is not connected to a communications network based on the prioritization rules; and, in a step 160, transmitting the first portion and the second portion of the received PPG data in the prioritized order when the PPG system is reconnected to the communications network.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one of a number or list of elements, and, optionally, additional unlisted items. Only terms clearly indicated to the contrary, such as "only one of' or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

As used herein, although the terms first, second, third, etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

Unless otherwise noted, when an element or component is said to be "connected to," "coupled to," or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of' and "consisting essentially of' shall be closed or semi-closed transitional phrases, respectively.

It should also be understood that, unless clearly indicated to the contrary, in any methods claimed herein that include more than one step or act, the order of the steps or acts of the method is not necessarily limited to the order in which the steps or acts of the method are recited.

The above-described examples of the described subject matter can be implemented in any of numerous ways. For example, some aspects can be implemented using hardware, software or a combination thereof. When any aspect is implemented at least in part in software, the software code can be executed on any suitable processor or collection of processors, whether provided in a single device or computer or distributed among multiple devices/computers.

The present disclosure can be implemented as a system, a method, and/or a computer program product at any possible technical detail level of integration. The computer program product can include a computer readable storage medium (or media) having computer readable program instructions thereon for causing a processor to carry out aspects of the present disclosure.

The computer readable storage medium can be a tangible device that can retain and store instructions for use by an instruction execution device. The computer readable storage medium can be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium comprises the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network can comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present disclosure can be assembler instructions, instruction-set-architecture (ISA) instructions, machine instructions, machine dependent instructions, microcode, firmware instructions, state-setting data, configuration data for integrated circuitry, or either source code or object code written in any combination of one or more programming languages, comprising an object oriented programming language such as Smalltalk, C++, or the like, and procedural programming languages, such as the "C" programming language or similar programming languages. The computer readable program instructions can execute entirely on the user's computer, partly on the user's computer, as a stand-alone software package, partly on the user's computer and partly on a remote computer or entirely on the remote computer or server. In the latter scenario, the remote computer can be connected to the user's computer through any type of network, comprising a local area network (LAN) or a wide area network (WAN), or the connection can be made to an external computer (for example, through the Internet using an Internet Service Provider). In some examples, electronic circuitry comprising, for example, programmable logic circuitry, field-programmable gate arrays (FPGA), or programmable logic arrays (PLA) can execute the computer readable program instructions by utilizing state information of the computer readable program instructions to personalize the electronic circuitry, in order to perform aspects of the present disclosure.

Aspects of the present disclosure are described herein with reference to flowchart illustrations and/or block diagrams of methods, apparatus (systems), and computer program products according to examples of the disclosure. It will be understood that each block of the flowchart illustrations and/or block diagrams, and combinations of blocks in the flowchart illustrations and/or block diagrams, can be implemented by computer readable program instructions.

The computer readable program instructions can be provided to a processor of a, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions can also be stored in a computer readable storage medium that can direct a computer, a programmable data processing apparatus, and/or other devices to function in a particular manner, such that the computer readable storage medium having instructions stored therein comprises an article of manufacture comprising instructions which implement aspects of the function/act specified in the flowchart and/or block diagram or blocks.

The computer readable program instructions can also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various examples of the present disclosure. In this regard, each block in the flowchart or block diagrams can represent a module, segment, or portion of instructions, which comprises one or more executable instructions for implementing the specified logical function(s). In some alternative implementations, the functions noted in the blocks can occur out of the order noted in the Figures. For example, two blocks shown in succession can, in fact, be executed substantially concurrently, or the blocks can sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

Other implementations are within the scope of the following claims and other claims to which the applicant can be entitled.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

## Claims

1. A method (100) for prioritizing transmission of physiological data, comprising:
providing (110) a mobile physiological data telemetry system (200), comprising a sensor (202) configured to receive physiological data from a patient, and further comprising a communication interface (204) configured to connect to a communications network, wherein the mobile physiological data telemetry system comprises a set of prioritization rules (206) for the patient;
receiving (120), from the sensor, physiological data from the patient;
determining (130), by the mobile physiological data telemetry system, that a first portion of the received physiological data is identified for transmission via the communication interface;
determining (140), by the mobile physiological data telemetry system, that a second portion of the received physiological data is identified for transmission via the communication interface;
prioritizing (150), when the mobile physiological data telemetry system is not connected to the communications network, and using the set of prioritization rules, transmission of the first portion before the second portion of the received physiological data, or vice versa; and
transmitting (160), when the mobile physiological data telemetry system is connected to the communications network, the first portion and the second portion of the received physiological data in the prioritized order.

2. The method of claim 1, wherein the mobile physiological data telemetry system is a wireless physiological sensor device attached to the patient.

3. The method of claim 1, wherein the mobile physiological data telemetry system comprises a smartphone, the smartphone comprising the wireless communication interface.

4. The method of claim 1, wherein determining that the first portion of the received E physiological CG data and the second portion of the received physiological data are identified for transmission is based on a parameter of the received physiological data.

5. The method of claim 1, wherein prioritization using the set of prioritization rules is based on a parameter of the received physiological data.

6. The method of claim 1, further comprising the step of receiving, by the mobile physiological data telemetry system, the set of prioritization rules for the patient.

7. The method of claim 1, wherein the set of prioritization rules are specific to a particular patient.

8. The method of claim 1, further comprising the step of determining (140), using the set of prioritization rules, that a third portion of the received physiological data is identified for transmission via the communication interface;
wherein the step of prioritizing comprises prioritizing transmission of the first portion, the second portion, and the third portion in a predetermined order of priority; and
wherein the step of transmitting comprises transmitting the first portion, the second portion, and the third portion in the predetermined order of priority.

9. A mobile physiological data telemetry system (200) for prioritizing transmission of physiological monitoring data, comprising:
a sensor (202) configured to receive physiological data from a patient;
a wireless communication interface (204) configured to connect to a communications network;
a set of prioritization rules (206) for the patient;
a processor (208) configured to: (i) receive, from the sensor, physiological data from the patient; (ii) determine, by the mobile physiological data telemetry system, that a first portion of the received physiological data is identified for transmission via the communication interface; (iii) determine, by the mobile physiological data telemetry system, that a second portion of the received physiological data is identified for transmission via the communication interface; (iv) prioritize, when the mobile physiological data telemetry system is not connected to the communications network, and using the set of prioritization rules, transmission of the first portion before the second portion of the received physiological data, or vice versa; and (v) transmit, via the communication interface when the mobile physiological data telemetry system is connected to the communications network, the first portion and the second portion of the received physiological data in the prioritized order.

10. The system of claim 9, wherein the mobile physiological data telemetry system is a wireless physiological sensor device attached to the patient.

11. The system of claim 9, further comprising a smartphone, the smartphone comprising the wireless communication interface.

12. The system of claim 9, wherein determining using the set of prioritization rules that the first portion of the received physiological data and the second portion of the received physiological data are identified for transmission is based on a parameter of the received physiological data.

13. The system of claim 9, wherein prioritization using the set of prioritization rules is based on a parameter of the received physiological data.

14. The system of claim 9, wherein the set of prioritization rules are specific to a particular patient.

15. The system of claim 9, wherein the processor is further configured to determine, using the set of prioritization rules, that a third portion of the received physiological data is identified for transmission via the communication interface;
wherein prioritizing comprises prioritizing, based on the set of prioritization rules, transmission of the first portion, the second portion, and the third portion in a predetermined order of priority; and
wherein transmitting comprises transmitting the first portion, the second portion, and the third portion in the predetermined order of priority.
